(19) Europäisches Patentamt
European Patent Office
Office européen des brevets

(11) **EP 4 205 770 A1**

(12) **EUROPÄISCHE PATENTANMELDUNG**

(43) Veröffentlichungstag:
**05.07.2023 Patentblatt 2023/27**

(21) Anmeldenummer: **21218354.5**

(22) Anmeldetag: **30.12.2021**

(51) Internationale Patentklassifikation (IPC):
**A61L 2/08** (2006.01)

(52) Gemeinsame Patentklassifikation (CPC):
**A61L 2/087**; A61L 2202/21

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**
Benannte Validierungsstaaten:
**KH MA MD TN**

(71) Anmelder: **Paul Hartmann AG
89522 Heidenheim (DE)**

(72) Erfinder: **Schmidt, Christian
73466 Lauchheim (DE)**

Bemerkungen:
Ein Antrag gemäss Regel 139 EPÜ auf Berichtigung
liegt vor. Über diesen Antrag wird im Laufe des
Verfahrens vor der Prüfungsabteilung eine
Entscheidung getroffen (Richtlinien für die Prüfung
im EPA, A-V, 3.).

(54) **IN-LINE STRAHLENSTERILISATION FÜR WUNDAUFLAGEN**

(57) Die vorliegende Erfindung betrifft ein in-line Verfahren zur Strahlensterilisation von Wundauflagen mit Elektronen. Eine verpackte Wundauflage wird mittels einer Produktionsanlage bereitgestellt und mittels einer Fördervorrichtung an ein Sterilisationsmodul weitergeleitet. Die verpackte Wundauflage wird mittels eines Sterilisationsmoduls, das in-line an die Produktionsanlage angekoppelt ist, sterilisiert. Das Sterilisationsmodule umfasst eine Elektronenstrahlquelle, welche Elektronen mit einer Energie zwischen 150 keV und 350 keV erzeugt. Die Energiedosis, welcher die verpackten Wundauflagen ausgesetzt sind, beträgt mindestens 15 kGy und maximal 30 kGy. Die Fördervorrichtung bewegt die verpackten Wundauflagen mit einer Geschwindigkeit von mindestens 10 m/min und höchstens 200 m/min.

Abbildung 2

**Beschreibung**

Die vorliegende Erfindung betrifft ein in-line Verfahren zur Strahlensterilisation von Wundauflagen mit Elektronen.

Derzeit wird die Sterilisation von Wundauflagen in der Regel mittels Ethylenoxid durchgeführt. Hierzu werden die einzelverpackten Wundauflagen zunächst kartoniert und palettiert. Nach Vorkonditionierung und Sterilisation muss das Sterilisationsgut ausreichend belüftet werden, um Rückstände von Ethylenoxid zu entfernen. Durch seine Komplexität ist dieses Verfahren aufwendig und kostspielig. Hinzu kommt, dass die Sterilisation von Wundauflagen üblicherweise in Sterilisationszentren durchgeführt wird, so dass Kosten für Transport von und zu diesen Sterilisationszentren entstehen. Insbesondere ist der Prozess von Versand über Vorkonditionierung, Sterilisation, Entgasung, Quarantäne bis zum Rückerhalt der Ware sehr zeitintensiv. Zykluszeiten von 10 Tagen sind üblich.

Alternativ ist eine Sterilisation mittels Strahlensterilisation unter Verwendung von Gammastrahlung möglich. Hierzu werden eine Vielzahl von in Kartons verpackten Wundauflagen sterilisiert, die sich auf Paletten befinden. Eine derartige Strahlensterilisation von Wundauflagen in palettierten Kartons mit Gammastrahlung ist bei Raumtemperatur möglich, jedoch benötigten die Sterilisationsvorrichtungen eine notwendige Abschirmung der Gammastrahlung. Das Verfahren benötigt als Strahlungsquelle radioaktives Cobalt 60. Dessen eingeschränkte Verfügbarkeit und die aufwendige Handhabung machen dieses Verfahren sehr kostspielig.

Im Stand der Technik wird Elektronenstrahlsterilisation für die Verpackung von chirurgischen und pharmazeutischen Produkten verwendet. In WO2009139073 wird ein Sterilisationsverfahren mittels Elektronenstrahl beschrieben, bei dem die Strahlung nur eine vergleichsweise geringe Eindringtiefe hat. Hierbei soll die Strahlung die Innenseite des Verpackungsmaterials erreichen, während gleichzeitig der Inhalt der Verpackung vor der schädlichen Wirkung der Strahlung geschützt wird.

Die WO02066081 betrifft ein Elektronenstrahl-Bestrahlungsgerät zum Sterilisieren eines bahnförmigen Verpackungsmaterials. Das Material wird von zwei Seiten mit einem Elektronenstrahl mit einer Energie von maximal 200 keV bestrahlt.

Die im Stand der Technik vorhandenen Erfindungen zu Strahlensterilisation betreffen insbesondere die Sterilisation von Verpackungen.

Weiterhin werden im Stand der Technik hochenergetische Elektronen in einem Energiebereich von 7 MeV bis 10 MeV verwendet, um sekundär (beispielsweise in einer Faltschachtel) bzw. tertiär (in einem Versandkarton) verpackte Medizinprodukte zu sterilisieren. Die hohe Energie ist erforderlich, um die gesamte Verpackung durchstrahlen zu können. Einrichtungen, die mit Elektronen in derart hohen Energiebereichen arbeiten, stellen sehr hohe Anforderungen an die Abschirmung und sind mit den üblichen Gegebenheiten in einer Produktionsanlage für Wundauflagen nicht oder nur sehr schwer vereinbar.

Es besteht ein Bedarf nach einem zuverlässigen Sterilisationsverfahren für Wundauflagen, welches am Ort der Produktion durchgeführt werden kann.

Die Erfinder der vorliegenden Erfindung standen vor der Aufgabe die Bereitstellung von sterilen Wundauflagen zu verbessern. Insbesondere standen die Erfinder der vorliegenden Erfindung vor der Aufgabe Wundauflagen, welche sich bereits in einer versiegelten Verpackung befinden, mittels ionisierender Strahlen zu sterilisieren. Hierbei waren aufgrund der bei Wundauflagen verwendeten Materialien und Verpackungen (Sterilbarrieresystem) spezielle Anforderungen zu berücksichtigen. Des Weiteren liegt der vorliegenden Erfindung die Aufgabe zugrunde, ein Sterilisationsverfahren bereitzustellen, welches sich besonders für in einer Primärverpackung verpackte Wundauflagen eignet.

Die Erfindung löst die Aufgabe mittels eines in-line Sterilisationsverfahrens für Wundauflagen nach Anspruch 1. Das Verfahren gewährleistet eine effiziente in-line Sterilisierung der verpackten Wundauflage, die mit den Vorgaben gemäß ISO 11137 übereinstimmt. Das Verfahren eignet sich insbesondere für in einer Primärverpackung versiegelte Wundauflagen. Unter dem Ausdruck "in-line Sterilisierung" wird vorliegend ein Sterilisationsprozess verstanden, welcher unmittelbar in der Produktionsanlage für die Wundauflage kontinuierlich abläuft oder an einem mit dieser Produktionsanlage funktionell verbundenen Sterilisationsmodul kontinuierlich durchgeführt wird. Die Sterilisation ist an den Herstellungsvorgang der Wundauflage gekoppelt. "In-line" bezeichnet somit eine zeitliche und räumliche Kopplung. Das Sterilisationsmodul kann als Komponente einer Fertigungsline verstanden werden. Der Durchsatz des Sterilisationsmoduls entspricht dann dem Durchsatz der Fertigungslinie. Die Fertigungslinie umfasst alle Maschinen bzw. Module, die zur Fertigung der verpackten sterilen Wundauflage beitragen. Diese Module der Fertigungslinie umfassen unter anderem ein oder mehrere Produktionsmodule (d.h. Maschinen, die zur Fertigung einer vollständigen Wundauflage aus ihren Ausgangsmaterialien oder aus einem Zwischenprodukt erforderlich sind), eine Verpackungsmaschine (d.h. eine Maschine, welche die Wundauflagen versiegelt, d.h. verpackt), ein Sterilisationsmodul (d.h. eine Vorrichtung, in welcher die verpackten Wundauflagen sterilisiert werden) und eine Fördervorrichtung. Unter der Bezeichnung "Produktionsanlage" wird vorliegend eine Vorrichtung verstanden, welche das Produktionsmodul und die Verpackungsmaschine umfasst.

Das Verfahren umfasst folgende Schritte:

- Bereitstellen der verpackten Wundauflage mittels einer Produktionsanlage
- Weiterleiten der verpackten Wundauflagen mittels einer Fördervorrichtung an ein Sterilisationsmodul,

- Sterilisieren der verpackten Wundauflage mittels mindestens einer in dem Sterilisationsmodul vorhandenen Elektronenstrahlquelle, wobei das Sterilisationsmodul in-line an die Produktionsanlage angekoppelt ist,

> a) und wobei die Energiedosis, welcher die verpackten Wundauflagen ausgesetzt sind, mindestens 15 kGy und maximal 30 kGy beträgt,
> b) und wobei die Energie der Elektronenstrahlung zwischen 150 keV und 350 keV liegt,
> c) und wobei die Fördervorrichtung die verpackten Wundauflagen mit einer Geschwindigkeit von mindestens 10 m/min und höchstens 200 m/min bewegt.

Bei der Produktionsanlage handelt es sich um eine vorzugsweise automatisch arbeitende Maschinenanordnung, auf welcher Wundauflagen unter Verwendung geeigneter Ausgangsmaterialien hergestellt werden können. Die Produktionsanlage umfasst beispielsweise Produktionsmodule, welche ausgestaltet sind, die üblicherweise in Materialbahnen vorliegenden Ausgangsmaterialien zuzuschneiden und zu laminieren. Die Produktionsanlage umfasst gleichfalls eine vorzugsweise automatisch arbeitende Verpackungsmaschine, die in dem Produktionsprozess denjenigen Modulen, welche die Wundauflagen herstellen, nachgeschaltet ist. Es wird eine Verpackungsmaschine eingesetzt, welche insbesondere für Sterilverpackungen besonders geeignet ist. Im Herstellungsprozess sind die Wundauflagen, welche durch die Verpackungsmaschine keimdicht eingepackt werden, üblicherweise zunächst unsteril, da der gesamte Herstellungsprozess in einer unsterilen Umgebung abläuft und auch die Ausgangsmaterialien nicht sterilisiert bereitgestellt werden.

Die von einem Produktionsmodul gefertigten Wundauflagen umfassen beispielsweise im Falle einer Inselwundauflage eine Wundkontaktschicht, eine absorbierende Lage (beispielsweise ein absorbierendes Kissen) und eine Abdeckschicht ("Backing"). Hierbei weist die Wundkontaktschicht eine kleinere Auflagefläche auf als die Abdeckschicht, wobei die Wundkontaktschicht entlang ihres Umfangs von der Abdeckschicht umgeben ist. Dabei kann die Abdeckschicht wundseitig eine adhäsive Beschichtung aufweisen, so dass die Wundauflage auf der Haut eines Patienten haften oder kleben kann. Eine weitere Ausführungsform der Wundauflage kann eine Wundkontaktschicht sein, bei der keine weiteren Lagen vorhanden sind. Wundauflagen, insbesondere solche mit adhäsiven Abdeckschichten oder Kleberändern, können weitere Materialien und Schichten zur Abdeckung der adhäsiven Bereiche oder zur rückseitigen Stabilisierung des Produkts, beispielsweise in Form von Trennfolien ("Liner") oder Stützfolien beinhalten. In Wundauflagen vorhandene Materialien umfassen beispielsweise Polyethylen (PE), Polypropylen (PP), Polyethylenterephthalat (PET), Polyurethan (PU), Viskose, Baumwolle, Silikon, Polyacrylate, Hydrokolloide oder Salbenmassen. Bei einer Sterilisation auf Basis von Elektronenstrahlen ist zu beachten, dass sich die Materialeigenschaften (beispielsweise Adhäsion, Farbe, Geruch, Zugfestigkeit) unter der Einwirkung der Bestrahlung verändern können.

Im Fertigungsprozess folgt auf die Herstellung der Wundauflagen die Verpackung der Wundauflagen. In der Verpackungsmaschine werden eine oder mehrere Wundauflagen in eine flexible Tasche oder in einen Beutel verpackt. Vorzugsweise wird nur eine einzige Wundauflage in je eine flexible Tasche oder je einen Beutel verpackt. Bei der Verpackungsmaschine kann es sich beispielsweise um Systeme handeln, wie in der bisher unveröffentlichten, Europäischen Patentanmeldung EP20201966.7 beschrieben. Eine Ausführungsform der Verpackung sind Peel-Beutel, welche aus einer oberen Sterilbarriere und einer unteren Sterilbarriere bestehen. Die Sterilbarrieren sind über einen anhaftenden Außenbereich verbunden, um einen Innenhohlraum zur Aufnahme von Wundauflagen zu bilden. Die obere und die untere Sterilbarriere besteht aus Papier, Folie oder einem Verbundstoff, wobei sich das Material der oberen Sterilbarriere von dem Material der unteren Sterilbarriere unterscheiden kann.

Ein weiteres Beispiel für Sterilverpackungen können thermogeformte Verpackungen sein.

In einer bevorzugten Ausführungsform befinden sich die Verpackungen in einer zusammenhängenden Beutelkette, wenn sie die Verpackungsmaschine verlassen. Die Verpackungen sind also endständig miteinander verbunden und müssen daher in einem späteren Prozessschritt zugeschnitten werden, um einzelne Verpackungen zu erhalten. Die zusammenhängenden Verpackungen und die darin eingeschlossenen Wundauflagen können eine Bahnbreite $b_W$ zwischen 0,02 m und 0,40 m haben. Bevorzugt sind Bahnbreiten $b_W$ zwischen 0,05 m und 0,35 m, besonders bevorzugt sind Bahnbreiten $b_W$ zwischen 0,1 m und 0,23 m. Die maximale Höhe der verpackten Wundauflagen beträgt bevorzugt zwischen 0,5 mm und 10,0 mm. Bevorzugter sind Höhen zwischen 1,0 mm und 5,0 mm. Besonders bevorzugt sind Höhen zwischen 1,5 und 2,5 mm. Das Flächengewicht der zusammenhängenden, verpackten Wundauflagen beträgt mindestens 0,15 kg/m$^2$ und höchstens 0,65 kg/m$^2$. Bevorzugt ist das durchschnittliche Flächengewicht der zusammenhängenden, verpackten Wundauflagen mindestens 0,2 kg/m$^2$ und höchstens 0,5 kg/m$^2$, bevorzugter zwischen 0,2 kg/m$^2$ und 0,45 kg/m$^2$ und besonders bevorzugt zwischen 0,2 kg/m$^2$ und 0,35 kg/m$^2$. Das Flächengewicht kann aus dem bekannten Flächengewicht der einzelnen Komponenten der Wundauflagen und ihrer Verpackung bestimmt werden.

Mittels der Fördervorrichtung wird die zusammenhängende Beutelkette über angetriebene und nicht angetriebene Walzen und/oder Förderbänder mit oder ohne Vakuumunterstützung zum Sterilisationsmodul befördert.

In einer anderen bevorzugten Ausführungsform wird die zusammenhängende Beutelkette vor dem Sterilisationsprozess in einzelne, getrennte Verpackungen, die je eine Wundauflage beinhalten, zugeschnitten. Die einzeln verpackten Wundauflagen werden dann an das Sterilisationsmodul weitergeleitet. Die verpackten Wundauflagen können auch mittels Unterdrucks an einem Vakuumband der Fördervorrichtung gehalten werden. Dabei ist die Materialauswahl des Vakuumförderbandes im Bereich des Sterilisationsmoduls auf eine dauerhafte Bestrahlung mit Elektronen der ausgewählten Energie und Menge abgestimmt. In einer bevorzugten Ausführungsform handelt es sich um ein Kettengliederband aus Metall. Diese Ausführungsform ist besonders vorteilhaft, da sie eine sehr hohe Stabilität gegenüber Strahlung aufweist und durch Sekundärelektronen zur Sterilisation beitragen kann. Zudem weist ein Kettengliederband aus Metall gute Wärmeleiteigenschaften auf. Die Beutel mit den darin befindlichen Wundauflagen werden mittels des Vakuumförderbands sicher in Position gehalten, während sie durch das Sterilisationsmodul transportiert werden. Danach können die Beutel vorzugsweise an ein weiteres, nachfolgend angeordnetes Modul übergeben werden. In einer möglichen Ausführungsform handelt es sich dabei um eine weitere Verpackungsmaschine, welche die einzelnen Beutel stapelt und in einer Faltschachtel (Sekundärverpackung) verpackt.

In einer anderen Ausführungsform werden die verpackten Wundauflagen nach Vereinzelung der Beutel mittels einer Fördervorrichtung, welche quer zur Transportrichtung verlaufende Rippen oder Erhebungen aufweist, transportiert. Zwischen zwei Erhebungen wird eine verpackte Wundauflage bewegt. Die Erhebungen halten die Wundauflagen während des Transports in den dafür vorhergesehenen Abschnitten der Fördervorrichtung.

In einer alternativen bevorzugten Ausführungsform können die einzeln verpackten Wundauflagen mittels Taschen, welche verschlossen oder teilweise geöffnet sind, auf der Fördervorrichtung gehalten werden.

Die Fördervorrichtung bewegt die verpackten Wundauflagen mit einer Geschwindigkeit von mindestens $v_W$ = 10 m/min und höchstens $v_W$ = 200 m/min. Bevorzugter bewegt die Fördervorrichtung die Wundauflagen mit einer Geschwindigkeit von $v_W$ = 20 m/min bis $v_W$ = 200 m/min. Besonders bevorzugt bewegt die Fördervorrichtung die Wundauflagen mit einer Geschwindigkeit von $v_W$ = 50 m/min bis $v_W$ = 200 m/min. Am bevorzugtesten bewegt die Fördervorrichtung die Wundauflagen mit einer Geschwindigkeit von $v_W$ = 100 m/min bis $v_W$ = 200 m/min.

Werden die verpackten Wundauflagen mittels der Fördervorrichtung in einer zusammenhängenden Beutelkette transportiert, so ist die Flächengeschwindigkeit $v_F$ = $v_W \cdot b_W$ mit der Geschwindigkeit $v_W$ und der Breite der Verpackung $b_W$. Die Breite der Verpackung $b_W$ ist senkrecht zur Förderrichtung definiert. Die Flächengeschwindigkeit kann in $^{m2}/_{min}$ angegeben werden. Die pro Minute bewegte Masse ist $v_m = \rho_G \cdot v_F$ mit dem Quadratmetergewicht $\rho_G$ der verpackten Wundauflage in einer zusammenhängenden Beutelkette in $^{kg}/_{m2}$ und $[v_m] = {}^{kg}/_{min}$. Die Flächengeschwindigkeit beträgt typischerweise zwischen $0,2 \frac{m^2}{min}$ und $80 \frac{m^2}{min}$, während die pro Sekunde bewegte Masse üblicherweise durch mindestens $0,03 \frac{kg}{min}$ und maximal $52 \frac{kg}{min}$ beschränkt ist.

In einer alternativen Ausführungsform werden separierte Beutel anstelle der zusammenhängen Beutelkette transportiert, wobei üblicherweise zwischen den Beuteln ein Abstand vom mindestens 1 mm vorhanden ist. Um einen im Vergleich zu der vorangehend beschriebenen Ausführungsform (zusammenhängenden Beutelkette) vergleichbare Produktionsgeschwindigkeit zu erhalten, muss die Geschwindigkeit der Fördervorrichtung erhöht werden. Bei einer erhöhten Geschwindigkeit der Fördervorrichtung muss jedoch die Leistung der Elektronenstrahlquelle erhöht werden, da Elektronen, die zwischen den Beuteln auftreffen, nicht zur Sterilisation beitragen.

Das Sterilisationsmodul umfasst mindestens eine Elektronenstrahlquelle und vorzugsweise mindestens eine Abschirmvorrichtung. Des Weiteren um umfasst das Sterilisationsmodul bevorzugt mindestens eine Führung für die Fördervorrichtung.

Bei der Elektronenstrahlquelle handelt es sich bevorzugt um einen Elektronenbeschleuniger. Im Elektronenbeschleuniger werden aus einer Elektronenquelle bevorzugt durch Glühemission aus einer Kathode mit bevorzugt niedriger Austrittsarbeit Elektronen freisetzt und diese durch elektrische Spannungen beschleunigt. Der Elektronenbeschleuniger und die Elektronenstrahlquelle können weitere Einheiten enthalten, die zur Konditionierung oder Lenkung des Elektronenstrahls geeignet sind.

In einer bevorzugten Ausführungsform ist das Sterilisationsmodul selbstabschirmend, so dass in dem Sterilisationsmodul vorhandene Bauteile wie beispielsweise die Walzen der Fördervorrichtung und/oder Gehäusewände und/oder weitere Bauteile einen ausreichenden Strahlenschutz gewährleisten, ohne dass weitere außerhalb des Sterilisationsmoduls anzubringende Abschirmungen erforderlich sind. Ein derartiges selbstabschirmendes Sterilisationsmodul lässt sich insbesondere bei der Verwendung von Elektronensterilisatoren, welche mit niedrigeren Elektronenenergien (beispielsweise bei 250 keV) arbeiten, besonders vorteilhaft umzusetzen.

Das Sterilisationsmodul ist bevorzugt in Verlängerung einer Geraden entlang der Transportrichtung der in der Fertigungslinie zwischen Produktionsmodul und Verpackungsmaschine vorhandenen Fördervorrichtung ausgerichtet. In einer anderen Ausführungsform ist das Sterilisationsmodul orthogonal zu dieser Transportrichtung ausgerichtet. Dazu werden die verpackten Wundauflagen bevorzugt in zwei Schritten um jeweils 45° umgelenkt und durch das Sterilisationsmodul geführt. Nach dem Sterilisationsmodul werden die Wundauflagen optional wieder so umgelenkt, dass sie wieder entlang der ursprünglichen Transportrichtung befördert werden. Wird eine zusammenhängenden Beutelkette bewegt, so werden dazu bevorzugt zwei Umlaufstangen verwendet, die im 22,5°-Winkel zu der Bahnrichtung stehen.

Gemäß DIN EN ISO 11137-1:2020-04 wird die von den verpackten Wundauflagen empfangene Energiedosis von 15 kGy als sicher für Medizinprodukte angesehen. Als obere Schranke für die Energiedosis können 30 kGy angesehen werden. Die obere Schranke ergibt sich aus Gründen der Wirtschaftlichkeit sowie in vielen Fällen aus der Materialverträglichkeit.

Bei Wundauflagen zeigen sich insbesondere Kleber empfindlich gegenüber zu hohen Energiedosen, bei denen sie ihre Klebkraft verlieren können. Andere Beispiele für Unverträglichkeiten gegenüber Energiedosen höher als 30 kGy können Verfärbungen von Kunststoffen oder einsetzende Brüchigkeit von Verpackungsmaterialien sein.

Die Erfinder der vorliegenden Erfindung haben herausgefunden, dass eine Energie $E$ zwischen 150 keV und 350 keV für die Bestrahlung von Wundauflagen besonders geeignet ist. Eine untere Grenze dieses Energiebereichs ergibt sich vorteilhaft aufgrund der Absorption der Strahlung in der verpackten Wundauflage. Elektronen mit geringerer Energie scheinen nicht tief genug in die verpackte Wundauflage einzudringen, um eine Sterilisationswirkung zu erreichen. Bis zu der erfindungsgemäß vorgeschlagenen unteren Grenze der Energie von 150 keV kann die unerwünscht starke Absorption der Strahlung in oberen Schichten, die zu einer ungleichmäßigen Energiedosisverteilung in der Wundauflage führt, kompensiert werden, indem die Anordnung des Sterilisationsmoduls so gewählt wird, dass die Produkte von beiden Seiten bestrahlt werden, beispielsweise durch zwei oder mehr aufeinanderfolgende Elektronenstrahlquellen auf gegenüberliegenden Seiten der Materialbahn.

Höhere Energien als 350 keV sind grundsätzlich zur in-line Sterilisation von Wundauflagen geeignet, jedoch nicht wirtschaftlich. Bei steigender Energie wird die mittlere freie Weglänge der Elektronen so groß, dass ein erheblicher Anteil der Strahlung die Wundauflage und Verpackung durchdringt, ohne absorbiert zu werden. Dieser Anteil trägt somit nicht zur Sterilisation bei. Elektronen mit einer Energie im Bereich von mehreren MeV, wie sie im Stand der Technik bereits erwähnt werden, führen zu einem erheblichen Aufwand bei der Erzeugung der Strahlung. Weiterhin erfordern Elektronenstrahlquellen, welche Elektronen mit einer sehr hohen Energie erzeugen, insbesondere solche welche eine Energie von wesentlich mehr als 350 keV erzeugen, aufwändige Abschirmungsmaßnahmen.

In einer weiteren möglichen Ausführungsform der Erfindung werden Energien von 280 keV bis 350 keV eingesetzt. Hierbei können diejenigen Elektronen, welche die Wundauflage ohne Wechselwirkung mit dem Material der Wundauflage durchdringenden, genutzt werden. Dies wird erreicht, indem die Materialbahn mehrfach, zum Beispiel in Schleifen, an der Elektronenstrahlquelle vorbeigeführt wird.

Die mindestens benötigte Leistung des Sterilisationsmoduls kann über $P_S = v_m \cdot D$ abgeschätzt werden. D ist die Energiedosis, die in $^{kJ}/_{kg}$ angegeben werden kann. Die Leistung $P_S$ beträgt üblicherweise mindestens 100 W und höchstens 100 kW. Bei der Bestimmung der tatsächlich benötigten Leistung sind neben Leistungsverlusten bei der Erzeugung des Elektronenstrahls vor allem zwei Effekte zu beachten: Zum einen ist von einer ungleichmäßigen Verteilung der Energiedosis innerhalb der Wundauflage und der Verpackung der Wundauflage auszugehen. Ein Indikator für eine ungleichmäßige Verteilung ist eine hohe dose uniformity ratio (DUR) nach ISO 11137-3:2017. Die DUR ist definiert als

$$\mathrm{DUR} = \frac{D_{\max}}{D_{\min}},$$

wobei $D_{\max}$ die maximale Energiedosis der absorbierten Energie innerhalb des Bestrahlungsbehälters ist und $D_{\min}$ die minimale Energiedosis der absorbierten Energie innerhalb des Bestrahlungsbehälters ist. Der Bestrahlungsbehälter ist die verpackte Wundauflage.

Da die niedrigste auf der sterilisierten Wundauflage gemessene Energiedosis für die Sterilisation ausreichen muss, kann es je nach Material und Höhe der verpackten Wundauflage zu hohen Energiedosen an anderen Stellen und somit insgesamt zu hohen DUR-Werten kommen. Die DUR liegt meist zwischen 1,1 und 10,0. Ein im Zusammenhang mit der Erfindung vorteilhafter DUR-Bereich liegt bei Werten zwischen 1,0 und 2,0 vor. Die weniger wünschenswerten hohen DUR-Werte treten vor allem bei niedrigen Energien auf. Insbesondere bei Produkten mit höherem Flächengewicht (beispielsweise Inselwundauflagen) sind zum Erreichen einer vorteilhaften DUR vergleichsweise hohe Energien, wie beispielsweise 280 keV, erforderlich.

Dies hat den weiteren Effekt, dass Bereiche des Produktes, insbesondere solche mit niedrigem Flächengewicht, bei diesen Energien bereits vollständig durchdrungen werden. Da die Elektronen ohne Wechselwirkung mit der Wundauflage die Wundauflage wieder verlassen, tragen diese Elektronen nicht zum Sterilisationsprozess bei und vermindern die Effizienz des Sterilisationsprozesses. In Experimenten ergaben sich Energiewerte von 250 keV als optimal für eine Wundauflage mit einem Flächengewicht von 200 g/m$^2$.

In einer bevorzugten Ausführungsform umfasst das Sterilisationsmodul eine Abschirmvorrichtung. Die Abschirmvorrichtung hält die von der Elektronenstrahlquelle abgegebene Strahlung von der Umgebung ab. Die Abschirmvorrichtung umgibt bevorzugt sowohl die Elektronenstrahlquelle als auch die Fördervorrichtung. Bevorzugt ist das Sterilisationsmodul selbstabschirmend, indem die Abschirmung durch Bauteile des Sterilisationsmoduls bewirkt wird. Die Abschirmung ist somit eine Komponente des Sterilisationsmoduls.

Nach einer vorteilhaften Ausführungsform umfasst die Abschirmvorrichtung Stahlplatten. Die Stahlplatten weisen vorzugsweise eine Höhe von 5 cm bis 32 cm, bevorzugt 10 cm bis 27 cm auf. Die Stahlplatten bilden bevorzugt einen zumindest teilweise abgeschlossenen Hohlkörper, in dessen Innenraum die Elektronenstrahlquelle eingebracht ist. Alternative Materialien hoher Dichte bzw. solche Materialien, welche Elementen mit hoher Ordnungszahl enthalten, gewährleisten gleichfalls eine effiziente Strahlenabschirmungen. Eine bevorzugte Ausführungsform stellen Abschirmungen aus Blei dar, die wesentlich dünner sein können als solche aus Stahl. Für Blei wird eine Abschirmung von bevorzugt 5 mm bis 80 mm Dicke verwendet, besonders bevorzugt 10 mm bis 54 mm.

Die verpackten Wundauflagen werden mittels der Fördervorrichtung durch die Abschirmvorrichtung zur Elektronenstrahlquelle geführt. Werden die verpackten Wundauflagen in der zusammenhängenden Beutelkette mittels der Fördervorrichtung transportiert, so können beispielsweise die Walzen der Fördervorrichtung Teil der Abschirmungsvorrichtung sein.

In einer bevorzugten Ausführungsform wird die zusammenhängende Beutelkette durch das Sterilisationsmodul mittels der Führung der Fördervorrichtung geführt. Die Führung der Fördervorrichtung umfasst im Bereich des Sterilisationsmoduls bevorzugt vier Walzen.

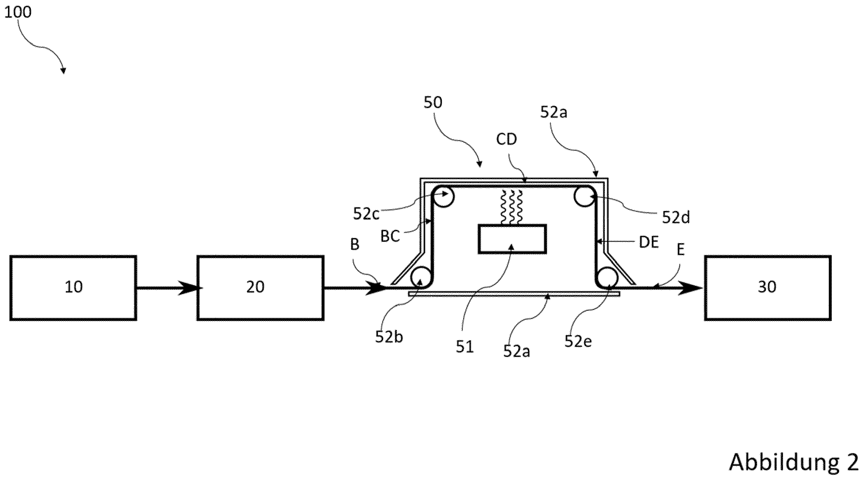

Über eine erste Walze wird die Beutelkette aus der ersten Ebene ausgelenkt. Die erste Ebene ist durch die in das Sterilisationsmodul einlaufende Beutelkette definiert. Mittels der zweiten und dritten Walze wird die Beutelkette in den Bereich der Elektronenstrahlquelle des Sterilisationsmoduls gelenkt. Mittels der von der Elektronenstrahlquelle ausgehenden Strahlung werden die verpackten Wundauflagen in der Beutelkette sterilisiert. Die vierte Walze lenkt die Beutelkette aus der zweiten Ebene nach Verlassen des Sterilisationsmoduls aus. Die zweite Ebene ist definiert durch die Ausrichtung der Beutelkette zwischen der dritten und vierten Walze. In dieser Ausführungsform werden die erste und vierte Walze als Teil der Abschirmvorrichtung verwendet. Diese Ausführungsform ist beispielhaft in Abbildung 2 dargestellt.

Die Bestrahlung von in einer Primärverpackung versiegelten Einzelprodukten, wie sie in der vorgestellten Erfindung vorgesehen ist, erweist sich als besonders vorteilhaft. Durch die im Vergleich zur Bestrahlung mehrerer Produkte in einer Sekundärverpackung deutlich verringerten Anzahl an Lagen in vertikaler Richtung kann auch bei niedrigen Energien eine gute Energiedosisverteilung erreicht werden, was sich vorteilhaft auf die Materialverträglichkeit auswirkt.

Aus der Energie der Elektronen ergibt sich die Beschleunigungsspannung des Sterilisationsmoduls $U_{\mathrm{S}} = \frac{E}{e}$ mit der Elementarladung e. Die für die Sterilisation benötigte Leistung kann von einer oder mehreren Elektronenstrahlquellen zur Verfügung gestellt werden.

Bevorzugt wird bei mehreren Elektronenstrahlquellen die Stromstärke variiert und die Beschleunigungsspannung konstant gehalten. Werden mehrere Elektronenstrahlquellen verwendet, so ist eine Bestrahlung der Wundauflage von zwei oder mehr Seiten denkbar.

Aufgrund der erfindungsgemäß verwendeten Energien kann es zur Sekundärstrahlung im Inneren der Wundauflage kommen. Besonders bei dünnen Materialien kann aufgrund von Rückstreuungen der primären Strahlung, die nicht von der Wundauflage absorbiert wurde, an dem Förderband Sekundärstrahlung auftreten. Daher kann es im Inneren der verpackten Wundauflagen zu einer höheren Energiedosis kommen als in dem zur Strahlenquelle hinzeigenden Oberflächenbereich.

Die Rückstreuung kann verwendet werden, um Wundauflagen mit geringer Flächendichte, (bevorzugt ist eine Flächendichte kleiner als 200 g/m²) mit Energien, welche insbesondere größer als 250 keV sind, effizient zu bestrahlen. Insbesondere eignen sich Wundauflagen mit homogenem Aufbau. In dieser Ausführungsform trifft die primäre Strahlung, die nicht von der Wundauflage absorbiert wurde auf einen metallischen Reflektor und wird von diesem zurückgestreut.

Die Fördervorrichtung wird mittels einer speicherprogrammierbaren Steuerung gesteuert und/oder geregelt. Beispielsweise kann die Fördervorrichtung abgeschaltet werden, wenn in die Steuerung ein Abschaltebefehl eingegeben wurde oder eine Beladung der Maschinen mit neuen Ausgangsmaterialien durchgeführt wird. In einer bevorzugten Ausführungsform ist die Steuerung der Fördervorrichtung an die Steuerung des Sterilisationsmoduls gekoppelt. Beispielsweise wird die Leistung der Elektronenstrahlquelle der Bahngeschwindigkeit angepasst.

Auch kann die Elektronenstrahlquelle dann beispielsweise im Falle einer Betriebsstörung der Produktionsanlage oder der Fördervorrichtung (beispielsweise ein Materialstau) schnell gestoppt werden.

Falls die verpackten Wundauflagen mittels der Fördervorrichtung in einer zusammenhängenden Beutelkette transportiert werden, so werden bei dieser Ausführungsform die zusammenhängenden Verpackungen der Wundauflagen nach der Sterilisation in einzeln verpackte Wundauflagen zerschnitten.

Messung der Energiedosis bei der Bestrahlung

Zur Bestimmung der tatsächlich bei der Sterilisation applizierten Energiedosis werden mehrere Dosimeter sowohl auf der Verpackung der Wundauflage als auch in der Verpackung der Wundauflage angebracht. Die in der Verpackung der Wundauflage eingebrachten Dosimeter befinden sich teilweise in der Wundauflage selbst und sind am weitesten von der Außenseite der Verpackung der Wundauflage entfernt.

Die Messwerte der auf der Außenseite der Verpackung der Wundauflage aufgebrachten Dosimeter dienen als Vergleichswerte. Sie werden nicht in der Berechnung der dose uniformity ratio (DUR) verwendet.

Eine DUR von 1 repräsentiert eine homogene Energiedosis über die gesamte verpackte Wundauflage. Eine gleichmäßige Bestrahlung über die gesamte verpackte Wundauflage ist erwünscht, daher stellt eine DUR nahe 1 ein anzustrebendes Ergebnis dar.

**Abbildungen**

Zum besseren Verständnis der vorliegenden Erfindung und ihrer Vorteile wird nun auf die folgenden Ausführungsformen in Verbindung mit den zugehörigen Abbildungen verwiesen.

Abbildung 1

Inselwundauflagen in zusammenhängenden Peel-Beuteln.

Abbildung 2

Schematischer Aufbau einer Ausführungsform der Anlage zur Herstellung einer sterilisierten, verpackten Wundauflage.

Abbildungen 3, 4, 5

Messwerte bei einer Beschleunigungsspannung von 200 keV, 250 keV und 280 keV.

Abbildung 6

Berechnete DUR für die Beschleunigungsspannungen 200 keV, 250 keV und 280 keV.

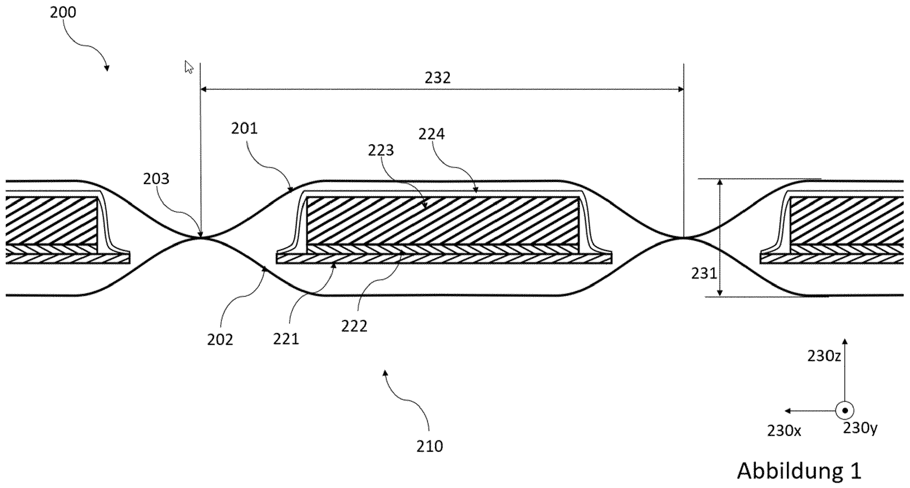

Abbildung 1 zeigt einen Querschnitt (nicht maßstabsgetreu) durch eine Kette verpackter Wundauflagen 200. Die Wundauflage 210 ist von einer ersten Materiallage 201 und einer zweiten Materiallage 202 eingeschlossen. Die erste Materiallage 201 und die zweite Materiallage 202 sind mittels einer Versiegelung 203 verbunden, so dass die erste Materiallage 201 und die zweite Materiallage 202 einen verschlossenen Verpackungsbeutel bilden, der die Wundauflage 220 beinhaltet. Eine einzelne verpackte Wundauflagen 210 ist an je gegenüberliegenden Seiten des Verpackungsbeutels mit je einer weiteren verpackten Wundauflage an der Versiegelung 203 verbunden. Die erste Materiallage 201 und die zweite Materiallage 202 erstrecken sich über die gesamte Länge aller verpackten Wundauflagen 200. Zusammen bilden alle verpackten Wundauflagen eine Beutelkette 200.

Die erste Materiallage 201 besteht aus einer transparenten Folie und die zweite Materiallage 202 besteht aus Papier.

Die Wundauflage 210 umfasst eine absorbierende Lage 223. Die absorbierende Lage 223 befindet sich zwischen der Abdeckschicht 224 und der Wundkontaktschicht 222. Die Wundkontaktschicht 222 ist durch ein Abziehpapier 221 vollständig bedeckt. Die Abdeckschicht 224 besteht beispielsweise aus einer Polyurethan-Folie. Die Wundkontaktschicht 222, welche eine kleinere Fläche als die Abdeckschicht 224 aufweist, kann beispielsweise aus einem hydrophilen Non-Woven Material oder aus einer mit einem adhäsiven Silikon beschichteten perforierten Polyurethanfolie gebildet sein.

Mittels einer Fördervorrichtung wird die zusammenhängende Beutelkette in Richtung 230x bewegt. Die Richtungen 230y und 230z sind senkrecht zu 230x und senkrecht zueinander definiert. Die Richtung 230y ist entlang der Breite der Wundauflage ausgerichtet und die Richtung 230z entlang der Höhe der Wundauflage.

Die Wundauflage mit Verpackung hat in dem gezeigten Beispiel eine maximale Höhe 231 von 2 mm. Die Länge 232 der verpackten Wundauflage ist bei diesem Beispiel 12 cm. Die einzelne, verpackte Wundauflage 210 ist 8 cm breit. Die unverpackte Wundauflage hat eine Breite von 5 cm und eine Länge von 7 cm. Die erste Materiallage 201 und die zweite Materiallage 202 sind 8 cm breit.

Die Länge der Wundauflage ist orthogonal zur Richtung 230x definiert und die Breite der Wundauflage ist entlang 230x definiert.

Abbildung 2 zeigt den schematischen Aufbau einer Anlage 100 für die Herstellung einer sterilisierten, verpackten Wundauflage. In dem Produktionsmodul 10 wird die Wundauflage hergestellt. Mittels einer Fördervorrichtung (hier nicht gezeigt) werden die Wundauflagen zu der Verpackungsmaschine 20 weitergeleitet und dort verpackt bzw. versiegelt, wobei die Verpackung eine Barriere für Keime bildet. Die Wundauflagen liegen nun in einer zusammenhängenden Beutelkette, wie in Abb. 1 gezeigt, vor. Die zusammenhängende Beutelkette wird zum Sterilisationsmodul 50 geleitet und in diesem durch die Walzen 52b, 52c, 52d, 52e zur Elektronenstrahlquelle, bei der es sich um den Elektronenbeschleuniger 51 handelt, geführt. Die Walzen 52b und 52e sind sowohl Teil der Abschirmvorrichtung als auch Teil der Fördervorrichtung.

Mittels einer ersten Walze 52b wird die Beutelkette aus der Ebene B ausgelenkt und verläuft dann in der Ebene BC. Die Ebene B ist durch die in das Sterilisationsmodul einlaufende Beutelkette definiert. Zwischen der ersten Walze 52b und der zweiten Walze 52c verläuft die Beutelkette in der Ebene BC. Mittels der zweiten Walze 52c und der dritten Walze 52d wird die Beutelkette in den Bereich der Elektronenstrahlquelle 51 des Sterilisationsmoduls 50 gelenkt und verläuft in der Ebene CD. Mittels der von der Elektronenstrahlquelle 51 ausgehenden Strahlung werden die verpackten Wundauflagen in der Beutelkette sterilisiert. Die vierte Walze 52e lenkt die Beutelkette aus der Ebene DE, welche durch die Ausrichtung der Beutelkette zwischen der dritten Walze 52d und der vierten Walze 52e gegeben ist, nach Verlassen des Sterilisationsmoduls 50 in die Ebene E aus. Die erste und vierte Walze 52b, 52e werden als Komponenten der Abschirmvorrichtung verwendet.

Eine weitere Abschirmung wird mittels der Abschirmvorrichtung 52a zur Verfügung gestellt. Die Abschirmvorrichtung 52a besteht aus Bleielementen von 54 mm Dicke. Nachdem die Beutelkette das Sterilisationsmodul 50 verlassen hat, wird sie zum Zuschneidemodul 30 gelenkt. Das Zuschneidemodul 30 zerschneidet die zusammenhängende Beutelkette in einzelne, verpackte Wundauflagen.

Bei der in Abbildung 2 gezeigten Ausführungsform eines Sterilisationsmoduls 50 werden Wundauflagen, welche eine maximalen Höhe von 2 mm aufweisen, bestrahlt. Die Geschwindigkeit der Fördervorrichtung beträgt 200 m/min. Die angelegte Beschleunigungsspannung der Elektronen beträgt 250 keV. Die Leistung der Elektronenstrahlquelle 51 beträgt 6,9 kW und die Stromstärke des Elektronenstrahls beträgt 27,6 mA. Der Sollwert der von der Wundauflage absorbierten Energiedosis beträgt 25 kGy.

Das in Abbildung 2 nur beispielhaft und zur Illustration einer möglichen Ausführungsform dargestellte Sterilisationsmodul 50 kann im Rahmen der vorliegenden Erfindung einen abweichenden Aufbau aufweisen. Ebenso kann die Bestrahlung der zu sterilisieren Wundauflagen im Rahmen der erfindungsgemäß vorgeschlagenen Bereiche mit unterschiedlichen Parametern durchgeführt werden. Insofern stellt die in Abbildung 2 gezeigte Ausführungsform nur eine von den Erfindern der vorliegenden Erfindung als vorteilhaft erkannte Variante dar.

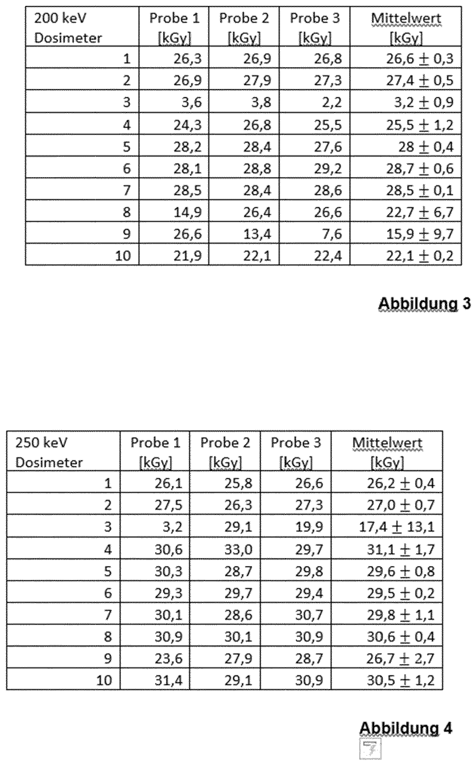

Die Messwerte, die in den Abbildungen 3 bis 5 wiedergegeben sind, wurden über die im Folgenden beschriebene Methode aufgenommen.

Je 2 Dosimeter sind auf der vorderen und hinteren Außenseite der Verpackung aufgebracht. Die Messwerte dieser Dosimeter 1, 2, 3, 4 gehen nicht in die DUR Berechnung ein. 1 und 2 sind auf der vorderen Außenseite befestigt und 3 und 4 auf der hinteren Außenseite. Die Dosimeter 5, 6, 7 sind auf der Innenseite der Verpackung zwischen vorderer Außenseite der Verpackung und der Wundauflage befestigt. Die Dosimeter 8 und 9 sind in der Verpackung und in die Wundauflage selbst eingebracht. Das Dosimeter 10 ist zwischen der hinteren Außenseite der Verpackung und der Wundauflage befestigt.

Die verpackte Wundauflage wird einseitig bestrahlt. Die Elektronenstrahlung trifft als erstes auf die vordere Außenseite, auf der die Dosimeter 1 und 2 aufgebracht sind.

Die Messungen werden bei einer Beschleunigungsspannung des Sterilisationsmoduls $U_S$ von 200 keV und einer Stromstärke von 5,6 mA, einer Beschleunigungsspannung von 250 keV und einer Stromstärke von 6,9 mA, sowie einer Beschleunigungsspannung von 280 keV und einer Stromstärke von 7,5 mA durchgeführt.

Der Sollwert der von der Wundauflage absorbierten Energiedosis beträgt 25 kGy.

Die verpackte Wundauflage hat eine Höhe von 2 mm.

Die verpackte Wundauflage wird von der Fördervorrichtung mit einer Geschwindigkeit von $v_w$ = 10 m/min durch den Bestrahlungsbereich des Sterilisationsmoduls befördert.

Für jede Beschleunigungsspannung werden 3 Messungen durchgeführt.

Um Rückstreuung zu vermeiden, werden die verpackten Wundauflagen auf ein 2 mm dickes Papier gelegt. Das Papier befindet sich auf der der Elektronenstrahlquelle abgewandten Seite.

Die Messergebnisse für die Beschleunigungsspannung von 200 keV werden in Abbildung 3 gezeigt. Die Messergebnisse für die Beschleunigungsspannung von 250 keV sind in Abbildung 4 dargestellt und die Messergebnisse für die Beschleunigungsspannung von 280 keV sind in Abbildung 5 zu finden.

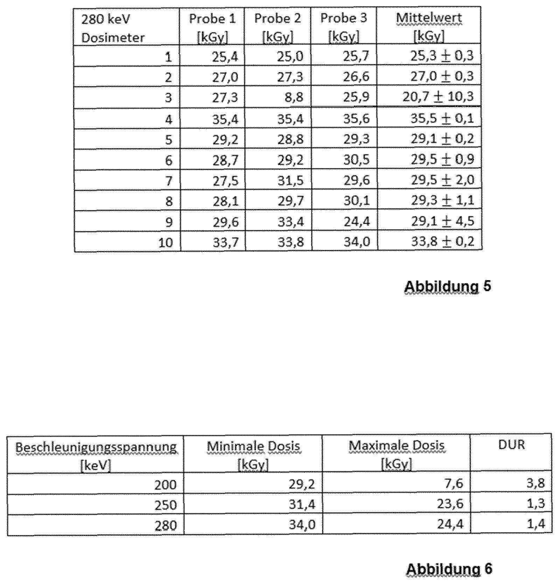

In Abbildung 6 wird die DUR zu allen Beschleunigungsspannungen berechnet. Aus allen Messungen, die zu den Dosimeter 5 bis 10 gehören, wird zu jeder Beschleunigungsspannung die höchste Energiedosis und die niedrigste Energiedosis ausgewählt. Danach wird die höchste Energiedosis durch die niedrigste Energiedosis geteilt, um den DUR-Wert zu erhalten

Für die Beschleunigungsspannung von 200 keV ergibt sich eine DUR von 3,83. Die Bestrahlung ist gleichmäßiger für die Beschleunigungsspannung von 280 keV. Die DUR liegt bei 1,39 für die Beschleunigungsspannung von 280 keV. Am gleichmäßigsten ist die Bestrahlung für die Beschleunigungsspannung von 250 keV. Die DUR beträgt 1,33 für die Beschleunigungsspannung von 250 keV.

Es zeigt sich, dass eine Beschleunigungsspannung von 250 keV besonders geeignet ist.

**Patentansprüche**

1.    Verfahren zur in-line Sterilisation einer verpackten Wundauflage, umfassend die folgenden Schritte

        - Bereitstellen der verpackten Wundauflage mittels einer Produktionsanlage (10, 20),
        - Weiterleiten der verpackten Wundauflage mittels einer Fördervorrichtung an ein Sterilisationsmodul (50),

- Sterilisieren der verpackten Wundauflage mittels mindestens einer in dem Sterilisationsmodul (50) vorhandenen Elektronenstrahlquelle (51), wobei das Sterilisationsmodul (50) in-line an die Produktionsanlage (10, 20) angekoppelt ist,

 a) und wobei die Energiedosis, welcher die verpackten Wundauflagen ausgesetzt sind, mindestens 15 kGy und maximal 30 kGy beträgt,

 b) und wobei die Energie der Elektronenstrahlung zwischen 150 keV und 350 keV liegt,

 c) und wobei die Fördervorrichtung die verpackten Wundauflagen mit einer Geschwindigkeit von mindestens 10 m/min und höchstens 200 min/m bewegt.

2. Verfahren nach Anspruch 1 **dadurch gekennzeichnet, dass** die verpackten Wundauflagen in endständig miteinander verbundenen Verpackungen an das Sterilisationsmodul (50) weitergeleitet werden und dass nach der Sterilisation die endständig miteinander verbundenen Verpackungen der Wundauflagen in einzeln verpackte Wundauflagen zugeschnitten werden.

3. Verfahren nach Anspruch 1 **dadurch gekennzeichnet, dass** die endständig miteinander verbundenen Verpackungen vor dem Sterilisieren in einzeln verpackte Wundauflagen zugeschnitten werden und die einzeln verpackten Wundauflagen an das Sterilisationsmodul (50) weitergeleitet werden.

4. Verfahren nach Anspruch 3 **dadurch gekennzeichnet, dass** die einzeln verpackten Wundauflagen mittels Unterdruck auf der Fördervorrichtung gehalten werden.

5. Verfahren nach Anspruch 3 **dadurch gekennzeichnet, dass** die einzeln verpackten Wundauflagen mittels Taschen, welche verschlossen oder teilweise geöffnet sind auf der Fördervorrichtung gehalten werden.

6. Verfahren nach mindestens einem der vorgenannten Ansprüche **dadurch gekennzeichnet, dass** das Sterilisationsmodul (50) selbstabschirmend ausgestaltet ist, indem die Abschirmung durch Bauteile des Sterilisationsmoduls (50) bewirkt wird.

7. Verfahren nach mindestens einem der vorgenannten Ansprüche **dadurch gekennzeichnet, dass** die Fördervorrichtung durch eine speicherprogrammierbare Steuerung gesteuert und/oder geregelt wird.

8. Verfahren nach mindestens einem der vorgenannten Ansprüche **dadurch gekennzeichnet, dass** die Wundauflage eine Höhe zwischen 0,5 mm und 10,0 mm aufweist.

9. Verfahren nach mindestens einem der vorgenannten Ansprüche **dadurch gekennzeichnet, dass** die verpackte Wundauflage ein durchschnittliches Flächengewicht zwischen 0,2 kg/m$^2$ und 0,5 kg/m$^2$ hat.

10. Verfahren nach mindestens einem der vorgenannten Ansprüche **dadurch gekennzeichnet, dass** es sich bei der Verpackung der verpackten Wundauflage um einen Peel-Beutel handelt, welcher aus einer ersten Sterilbarriere (201) und einer zweiten Sterilbarriere (202) besteht, wobei die erste Sterilbarriere (201) mit der zweiten Sterilbarriere (202) über einen anhaftenden Außenbereich (203) verbunden ist, um einen Innenhohlraum zur Aufnahme der Wundauflage zu bilden.

Abbildung 1

Abbildung 2

| 200 keV Dosimeter | Probe 1 [kGy] | Probe 2 [kGy] | Probe 3 [kGy] | Mittelwert [kGy] |
|---|---|---|---|---|
| 1 | 26,3 | 26,9 | 26,8 | 26,6 ± 0,3 |
| 2 | 26,9 | 27,9 | 27,3 | 27,4 ± 0,5 |
| 3 | 3,6 | 3,8 | 2,2 | 3,2 ± 0,9 |
| 4 | 24,3 | 26,8 | 25,5 | 25,5 ± 1,2 |
| 5 | 28,2 | 28,4 | 27,6 | 28 ± 0,4 |
| 6 | 28,1 | 28,8 | 29,2 | 28,7 ± 0,6 |
| 7 | 28,5 | 28,4 | 28,6 | 28,5 ± 0,1 |
| 8 | 14,9 | 26,4 | 26,6 | 22,7 ± 6,7 |
| 9 | 26,6 | 13,4 | 7,6 | 15,9 ± 9,7 |
| 10 | 21,9 | 22,1 | 22,4 | 22,1 ± 0,2 |

Abbildung 3

| 250 keV Dosimeter | Probe 1 [kGy] | Probe 2 [kGy] | Probe 3 [kGy] | Mittelwert [kGy] |
|---|---|---|---|---|
| 1 | 26,1 | 25,8 | 26,6 | 26,2 ± 0,4 |
| 2 | 27,5 | 26,3 | 27,3 | 27,0 ± 0,7 |
| 3 | 3,2 | 29,1 | 19,9 | 17,4 ± 13,1 |
| 4 | 30,6 | 33,0 | 29,7 | 31,1 ± 1,7 |
| 5 | 30,3 | 28,7 | 29,8 | 29,6 ± 0,8 |
| 6 | 29,3 | 29,7 | 29,4 | 29,5 ± 0,2 |
| 7 | 30,1 | 28,6 | 30,7 | 29,8 ± 1,1 |
| 8 | 30,9 | 30,1 | 30,9 | 30,6 ± 0,4 |
| 9 | 23,6 | 27,9 | 28,7 | 26,7 ± 2,7 |
| 10 | 31,4 | 29,1 | 30,9 | 30,5 ± 1,2 |

Abbildung 4

| 280 keV Dosimeter | Probe 1 [kGy] | Probe 2 [kGy] | Probe 3 [kGy] | Mittelwert [kGy] |
|---|---|---|---|---|
| 1 | 25,4 | 25,0 | 25,7 | 25,3 ± 0,3 |
| 2 | 27,0 | 27,3 | 26,6 | 27,0 ± 0,3 |
| 3 | 27,3 | 8,8 | 25,9 | 20,7 ± 10,3 |
| 4 | 35,4 | 35,4 | 35,6 | 35,5 ± 0,1 |
| 5 | 29,2 | 28,8 | 29,3 | 29,1 ± 0,2 |
| 6 | 28,7 | 29,2 | 30,5 | 29,5 ± 0,9 |
| 7 | 27,5 | 31,5 | 29,6 | 29,5 ± 2,0 |
| 8 | 28,1 | 29,7 | 30,1 | 29,3 ± 1,1 |
| 9 | 29,6 | 33,4 | 24,4 | 29,1 ± 4,5 |
| 10 | 33,7 | 33,8 | 34,0 | 33,8 ± 0,2 |

**Abbildung 5**

| Beschleunigungsspannung [keV] | Minimale Dosis [kGy] | Maximale Dosis [kGy] | DUR |
|---|---|---|---|
| 200 | 29,2 | 7,6 | 3,8 |
| 250 | 31,4 | 23,6 | 1,3 |
| 280 | 34,0 | 24,4 | 1,4 |

**Abbildung 6**

**Europäisches Patentamt**
**European Patent Office**
**Office européen des brevets**

# EUROPÄISCHER RECHERCHENBERICHT

**Nummer der Anmeldung**

EP 21 21 8354

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | KR 102 283 464 B1 (KYUNGPOOK NAT UNIV IND ACADEMIC COOP FOUND [KR] ET AL.) 2. August 2021 (2021-08-02) * Zusammenfassung * * Absatz [0073] * ----- | 1-10 | INV. A61L2/08 |
| A | WO 2018/036900 A1 (BÜHLER AG [CH]) 1. März 2018 (2018-03-01) * Ansprüche 1,2,4; Abbildung 2 * ----- | 1-10 | |
| A | US 10 301 052 B2 (FORSELL JAMES H [US]; COLE GERALD JOHN [US]; HOLIMAN JEFFREY DOW [US]) 28. Mai 2019 (2019-05-28) * Zusammenfassung * ----- | 1-10 | |
| A | CN 106 475 286 A (HIPRO POLYMER MAT (JIANGSU) CO LTD; NANJING INST TECHNOLOGY) 8. März 2017 (2017-03-08) * Zusammenfassung * ----- | 1-10 | |
| A | KR 2018 0006784 A (KUMOH NAT INST TECHNOLOGY IND ACAD COOP FOUND [KR]) 19. Januar 2018 (2018-01-19) * Zusammenfassung * ----- | 1 | RECHERCHIERTE SACHGEBIETE (IPC) A61L A61F |

Der vorliegende Recherchenbericht wurde für alle Patentansprüche erstellt

| Recherchenort | Abschlußdatum der Recherche | Prüfer |
|---|---|---|
| München | 3. Juni 2022 | Fischer, Michael |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

............................................................

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 21 21 8354

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

03-06-2022

| Im Recherchenbericht angeführtes Patentdokument | Datum der Veröffentlichung | Mitglied(er) der Patentfamilie | Datum der Veröffentlichung |
|---|---|---|---|
| KR 102283464 B1 | 02-08-2021 | KEINE | |
| WO 2018036900 A1 | 01-03-2018 | BR 112019003331 A2 | 04-06-2019 |
| | | CA 3036931 A1 | 01-03-2018 |
| | | CN 109640695 A | 16-04-2019 |
| | | EA 201990533 A1 | 28-06-2019 |
| | | EP 3284351 A1 | 21-02-2018 |
| | | ES 2722054 T3 | 07-08-2019 |
| | | JP 6646787 B2 | 14-02-2020 |
| | | JP 7044812 B2 | 30-03-2022 |
| | | JP 2019532622 A | 14-11-2019 |
| | | JP 2020078308 A | 28-05-2020 |
| | | PL 3284351 T3 | 30-08-2019 |
| | | US 2019183137 A1 | 20-06-2019 |
| | | US 2022000130 A1 | 06-01-2022 |
| | | WO 2018036900 A1 | 01-03-2018 |
| US 10301052 B2 | 28-05-2019 | KEINE | |
| CN 106475286 A | 08-03-2017 | KEINE | |
| KR 20180006784 A | 19-01-2018 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2009139073 A
- WO 02066081 A

- EP 20201966